# EUROPEAN PATENT SPECIFICATION

(11) **EP 2 219 564 B1**
(45) Date of publication and mention of the grant of the patent: **24.07.2019**
(21) Application number: 08864467.9
(22) Date of filing: 19.12.2008
(51) Int. Cl.: A61F 2/91, A61F 2/915

(54) **ENDOPROSTHESIS HAVING A STABLE ARCHITECTURE AND FLEXIBLE CONNECTORS**
ENDOPROTHESE MIT STABILER ARCHITEKTUR UND FLEXIBLEN VERBINDUNGSELEMENTEN
ENDOPROTHÈSE PRÉSENTANT UNE ARCHITECTURE STABLE ET ENDOPROTHÈSE PRÉSENTANT DES RACCORDS FLEXIBLES

(30) Priority: 20.12.2007 US 961290; 20.12.2007 US 961384
(43) Date of publication of application: 25.08.2010
(73) Proprietor: Abbott Laboratories Vascular Enterprises Limited, Dublin 2 (IE)
(72) Inventor: BREGULLA, Rainer, 72336 Balingen (DE); OSMAN, Karim, Mountain View CA 94040 (US); NEWHAUSER, Richard, Redwood City CA 94062 (US)
(74) Representative: Peters, Hajo
(86) International application number: PCT/EP2008/010953
(87) International publication number: WO 2009/080327

(56) References cited:
- EP-A- 0 983 753
- EP-A- 1 516 600
- WO-A-01/82835
- US-A1- 2004 230 293
- US-A1- 2006 106 452

## Description

### FIELD OF THE INVENTION

The present invention relates to an endoprosthesis having elevated scaffolding properties and also elevated flexibility. More particularly, the present invention relates to an endoprosthesis having a plurality of web rings coupled by connectors that are composed of five struts of essentially equal length, which extend circumferentially in essentially parallel directions. An endoprosthesis having elevated axial and torsional flexibility and improved resistance to clinical fatigue having a plurality of web rings coupled by connectors composed of sequentially adjoined struts with foot-shaped extensions protruding from the intersections of pairs of struts, is also described.

### BACKGROUND OF THE INVENTION

Applications of endoprotheses to the superior femoral artery (SFA) and to the popliteal artery (PA) has received increased attention because of the prevalence of peripheral arterial disease (PAD) among older patients and because no known endoprosthesis can adequately support the SFA and the PA without distorting its architecture during patient motion.

PAD is estimated to affect between 3% and 10% of individuals till the age of 70 and may approach 20% of individuals older than 70 years of age. PAD has been associated with an increased risk of coronary artery disease, cerebrovascular disease, and premature death. Moreover, as a consequence of limited exercise performance and walking ability, individuals who have symptoms of intermittent claudication experience a significantly negative impact on quality of life.

Exercise programs have been recommended as the first line of therapy for PAD. Pharmacotherapy with cilostazol provides additional symptom relief, but patients who fail medical therapy and continue to have resting leg pain or non-healing ulcers eventually become candidates for invasive treatment strategy. Unfortunately, surgical revascularization has associated with higher periprocedural morbidity and mortality, making the surgical option less desirable in elderly patients - a significant proportion of patients with PAD.

Because greater than 50% of individuals with lower extremity claudication have atherosclerotic disease confined to the superficial femoral artery (SFA), endovascular techniques have recently emerged to treat this arterial segment.

Stents, grafts and a variety of other endoprostheses are well known and used in endovascular procedures, such as for treating aneurysms, lining or repairing vessel walls, filtering or controlling fluid flow, and expanding or scaffolding occluded or collapsed vessels. Such endoprostheses can be delivered and used in virtually any accessible body lumen of a human or animal and can be deployed by any of a variety of recognized means.

An endoprosthesis is typically delivered by a catheter system to a desired location or deployment site inside a body lumen of a vessel or other tubular organ. To facilitate such delivery, the endoprosthesis must be capable of having a particularly small cross profile and a sufficient degree of longitudinal flexibility during delivery to allow advancement through the anatomy to the deployed site. Additionally, the intended deployment site may be difficult to access by a physician and often involves traversing the delivery system through the tortuous pathway of the anatomy. Therefore, it would be desirable to provide the endoprosthesis with a sufficient degree of longitudinal flexibility during delivery to allow advancement through the anatomy to the deployed site.

Once deployed, the endoprosthesis should be capable of satisfying a variety of performance characteristics. The endoprosthesis should have sufficient rigidity or outer bias to perform its intended function, such as opening a lumen or supporting a vessel wall. Similarly, the endoprosthesis should retain sufficient flexibility along its length in its expanded condition so that it will not kink, straighten or fracture during or after deployment in a curved vessel. The endoprosthesis should also provide a substantially uniform or otherwise controlled scaffolding of the vessel wall and prevent plaque from protruding into the artery.

One type of endoprosthesis is the stent, which is used for the treatment of atherosclerotic stenosis in blood vessels. After a patient undergoes a percutaneous transluminal angioplasty or similar interventional procedure, a stent may be deployed at the treatment site to maintain patency of the vessel. The stent is configured to scaffold or support the treated blood vessel and may be loaded with a beneficial agent, acting as a delivery platform to reduce restenosis or the like.

Numerous endoprosthesis designs and constructions have been developed to address one or more of the performance characteristics summarized above. For example, a variety of stent designs are disclosed in the following patents: U.S. Patent No. 4,580,568 to Gianturco; U.S. Patent No. 5,102,417 to Palmaz; U.S. Patent No. 5,104,404 to Wolff; U.S. Patent No. 5,133,732 to Wiktor; U.S. Patent No. 5,292,331 to Boneau; U.S. Patent No. 5,514,154 to Lau et al.; U.S. Patent No. 5,569,295 to Lam; U.S. Patent No. 5,707,386 to Schnepp-Pesch et al.; U.S. Patent No. 5,733,303 to Israel et al.; U.S. Patent No. 5,755,771 to Penn et al.; U.S. Patent No. 5,776,161 to Globerman; U.S. Patent No. 5,895,406 to Gray et al.; U.S. Patent No. 6,033,434 to Borghi; U.S. Patent No. 6,099,561 to Alt; U.S. Patent No. 6,106,548 to Roubin et al.; U.S. Patent No. 6,113,627 to Jang; U.S. Patent No. 6,132,460 to Thompson; U.S. Patent No. 6,331,189 to Wolinsky et al.; and U.S. Patent No. 7,128,756 to Lowe et al..

During use, an endoprosthesis is subjected to a variety of stresses and strains due to compressive, bending and torsional forces applied to the endoprosthesis. Current endoprosthesis designs provide only limited resistance to clinical fatigue, sometimes leading to stent fracture after implantation. This problem is particularly acute for endoprosthesis implanted in body portions that subject the endoprosthesis to severe environments, for example, for stents implants in the superficial femoral artery (SFA). A stent implanted in the SFA is subject to bending and torsional forces after implantation, which may cause the stent to eventually fracture. Not only does a fracture cause a loss of scaffolding properties of the stent and a possible puncture of the vessel, but clinical studies have shown a correlation between stent fracture and restenosis.

In addition, during the treatment of some types of SFA and PA disease, relatively long stent lengths are frequently required, at times causing the treating physician to overlap multiple stents. Further complicating the treatment of SFA and PA disease is, as mentioned above, the possibility of stent fractures and subsequent restenosis. A particular area of vulnerability is the area through the adductor canal as the SFA continues behind the knee; in fact, the area at the adductor canal is a frequent location for SFA disease likely secondary to the bending, compression, elongation and torsion forces on the artery itself due to the muscular structure surrounding this canal, leading to a lengthening and shortening of up to 15% of the stent between straight and bent positions of a limb. After the stent has been implanted, the body vessel is subjected to repeated traumas caused by the negative interaction of a relatively rigid stent and a softer artery.

In the earlier days of endovascular therapy, it was believed that the area to avoid stenting due to the risk of stent crush or stent fracture was near the bony articulation between the femur and the tibia. It is now believed that the area of critical importance is really superior to this point leading up to the adductor canal, which is a frequent location of SFA lesions. If it is necessary to stent this region, the ability of the stent to withstand the forces present in the SFA is of critical importance. Another risk is the incidence of restenosis, against which self-expanding Nitinol stents have shown better one-year patency rates than other types of stents.

Therefore, it would be desirable for the endoprosthesis to provide an elevated degree of scaffolding to a vessel wall while retaining an elevated degree of flexibility within the operating environments of the SFA and PA. Further, it would also be desirable to provide an endoprosthesis design that not only provides increased axial and torsional flexibility of the endoprosthesis, but that also offers improved resistance to clinical fatigue.

### SUMMARY OF THE INVENTION

The present invention relates to an endoprosthesis for delivery within a body lumen that provides an elevated degree of scaffolding and that has an elevated degree of flexibility, making it particularly suited for implantation in body vessels in which extensive bending, compression, elongation, and torsion forces are applied to the endoprosthesis. In different embodiments, the endoprosthesis may be configured as a stent, graft, valve, occlusive device, trocar or aneurysm treatment device for a variety of intralumenal applications, including vascular, coronary, biliary, esophageal, renal, urological and gastrointestinal, for example, for the treatment of SFA and PA diseases.

An endoprosthesis constructed according to the principles of the present invention includes a web structure that is expandable from a contracted configuration to an expanded configuration and that includes a plurality of longitudinally adjacent web rings. Each of the web rings is defined by web elements disposed circumferentially around a longitudinal axis, which are adjoined one to the other at junction bends. A first junction bend in a first web ring is coupled to a second junction bend in a second web ring by a connector that includes five struts of essentially equal length that extend circumferentially in essentially parallel directions. The struts of the connector are adjoined in sequence by coupling segments that, in one embodiment of the invention, are arcuate in shape.

The connector of the present invention also includes two struts of reduced length, one coupled to the first junction bend and the other one coupled to the second junction bend. To increase density of the endoprosthesis, the coupling segments of one connector may be nested among the coupling segments of another connector adjacent in a circumferential direction.

In different embodiments, the connector may couple a midpoint in the first junction bend to a midpoint in the second junction bend, or may couple an endpoint in the first junction bend to an endpoint in the second junction bend. The first junction bend may be laterally offset in relation to the second junction bend, providing the endoprosthesis with greater ability to absorb torsional stresses.

The connector may span circumferentially for a distance substantially equal to the circumferential spacing between the midpoints of four junction bends, and the interstices between the struts may be narrower than the widths of the struts.

In different embodiments, the endoprosthesis may be a stent, and the struts of the connector may be substantially rectilinear in shape. The web elements may also be substantially rectilinear in shape, or may be shaped like crowns that include a central member disposed essentially parallel to the longitudinal axis of the endoprosthesis in the contracted configuration and connected at its ends to end members that extend at obtuse angles from the central member. In the contracted delivery configuration of the endoprosthesis, the web elements of each web ring are nested one into the other and may be oriented at approximately 180 degrees in relation to the web elements in a neighboring web ring.

The web structure may be manufactured from a shape-memory material and may be configured to self-expand from the contracted configuration to the expanded configuration, or may be expanded by application of a radial pressure to an interior surface of the essentially tubular body, for example, by inflating a balloon disposed inside the endoprosthesis.

An endoprosthesis for delivery in a body lumen that includes a plurality of web rings coupled one to the other by flexible connectors is also described. Such connectors are structured to absorb at least some of the axial and torsional stresses applied to the endoprosthesis and to improve resistance of the endoprosthesis to clinical fatigue and are each composed of struts sequentially adjoined one to the other, with foot-shaped extensions protruding at the intersections of pairs of the struts.

An endoprosthesis not constructed according to the principles of the present invention includes a web structure expandable from a contracted delivery configuration to an expanded deployed configuration. The web structure is composed of a plurality of longitudinally adjacent web rings, which in turn are defined by a plurality of web elements. The web elements are disposed substantially parallel to the longitudinal axis of the tubular body when the stent is in the contracted delivery configuration, and pairs of the web elements are adjoined one to the other sequentially at junction bends. A connector couples a first junction bend in a first web ring to a second junction bend in a second web ring.

The connector is formed by a plurality of struts that are joined one to the other at intersections. A foot extension protrudes from at least some of the intersections between the struts, and includes an essentially rectilinear portion that provides the sole portion of the foot extension and an essentially arcuate portion that provides the toe portion of the foot extension.

In one example the plurality of struts forming the connectors are disposed transversally in relation to the longitudinal axis of the tubular body, while the sole portions of the foot extensions are disposed essentially parallel to the longitudinal axis. In a preferred example each connector is formed by three struts and two foot extensions. The first and third struts are disposed at an angle between about 95 and about 115 degrees in relation to the longitudinal axis and the third strut disposed at an angle between about 75 and about 85. A first foot extension couples the first and the second struts, and a second foot extension couples the second and the third struts.

The struts forming the connector may be rectilinear, multi-segment, or curvilinear, and may be of the same length or of different lengths. The foot extension nearer to the first web ring may be oriented towards the second web ring and the foot extension nearer to the second web ring may be oriented towards the first web ring, but in different embodiments, the foot extensions may be oriented in other directions.

In an example, the junction bends coupled by a connector are laterally offset one in relation to the other, and less than all junction bends in one web ring are coupled by connectors to junction bends in a neighboring ring. In this example, the connectors connecting a first web ring to a second web ring are also laterally offset and oriented symmetrically in relation to the connectors connecting the second web ring to a third web ring. Additionally, each connector may couple the midpoint of a first junction bend to the midpoint of a second junction bends, or couple a first point substantially at one end of a first junction bend to a second point substantially at one end of a second junction bend.

The endoprosthesis may also have web rings with web elements of different shapes. In one example, each of the web rings is defined by web elements composed of a central member disposed essentially parallel to the longitudinal axis of the endoprosthesis when in the contracted delivery configuration and coupled at each end to an end member to form a crown profile. The web elements of each web ring are nested one into the other when in the contracted delivery configuration and are oriented at approximately 180 degrees (that is, in opposite directions) in relation to the web elements of the neighboring web ring. In other embodiments, the web elements may have a variety of other configurations.

### BRIEF DESCRIPTION OF THE DRAWINGS

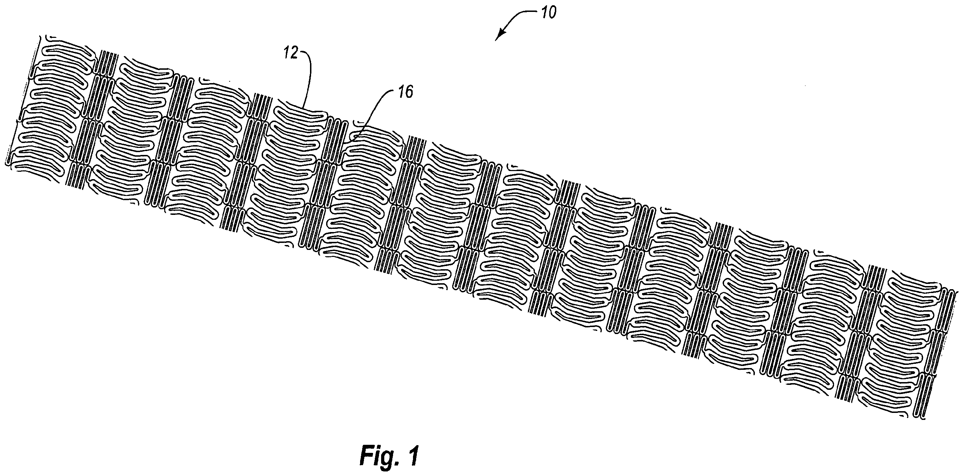

The drawings constitute a part of this specification and figures 1 to 6A include exemplary embodiments of the invention, which may be embodied in various forms. It is to be understood that in some instances various aspects of the invention may be shown exaggerated or enlarged to facilitate an understanding of the invention.
FIG. 1 is a top view of the web structure of a first endoprosthesis, illustrated in a flattened state, according to an embodiment of the invention.
FIG. 2 is a detail view of the web structure of FIG. 1.
FIG. 3 and FIG. 3A (the corresponding photo) illustrate an endoprosthesis according to an embodiment of the invention in an at rest state.
FIG. 4 and FIG. 4A (the corresponding photo) illustrate the endoprosthesis of FIG. 3 and FIG. 3A in a bent state.
FIG. 5 and FIG. 5A (the corresponding photo) illustrate the endoprosthesis of FIG. 3 and FIG. 3A in an elongated state.
FIG. 6 and FIG. 6A (the corresponding photo) illustrate the endoprosthesis of FIG. 3 and FIG. 3A in a twisted state.
FIG. 7 illustrates a perspective view of another endoprosthesis.
FIG. 8 illustrates a detail view, in flattened form, of the web structure of the embodiment of FIG. 7.
FIG. 9A illustrates a detail view of the web structure of FIG. 8, and FIG. 9B illustrates a detail view of the connector depicted in FIG. 9A.
FIG. 10 illustrates a detail view of a variant of the endoprosthesis illustrated in FIG. 9.
FIG. 11 illustrates a detail view, in flattened form, of a web structure according to another endoprosthesis.

### DETAILED DESCRIPTION OF EMBODIMENTS OF THE INVENTION

Detailed descriptions of embodiments of the invention are provided herein. It is to be understood, however, that the present invention may be embodied in various forms. Therefore, the specific details disclosed herein are not to be interpreted as limiting, but rather as a representative basis for teaching one skilled in the art how to use the present invention in virtually any detailed system, structure or manner.

The present invention relates to an endoprosthesis for delivery within a body lumen that provides an elevated degree of scaffolding and that has an elevated degree of flexibility, making it particularly suited for implantation in body vessels in which extensive bending, compression, elongation, and torsion forces are applied to the endoprosthesis. In different embodiments, the endoprosthesis may be configured as a stent, graft, valve, occlusive device, trocar or aneurysm treatment device for a variety of intralumenal applications, including vascular, coronary, biliary, esophageal, renal, urological and gastrointestinal, for example, for the treatment of SFA and PA diseases.

An endoprosthesis constructed according to the principles of the present invention includes a web structure that is expandable from a contracted delivery configuration to an expanded deployed configuration and that is formed by a plurality of longitudinally adjacent web rings, coupled by connectors that include five struts of essentially equal length. The connector struts are joined in sequence and extend circumferentially in essentially parallel directions. For ease of description and without restrictive intent, an embodiment of the invention will be described hereinafter with reference to a stent.

Referring first to FIG. 1, a first embodiment of the invention relates to a stent 10, which is formed by a plurality of web rings 12 that are disposed longitudinally one next to the other and that are adjoined by connectors 14 constructed as described in greater detail hereinbelow. Although the structure of stent 10 is shown in FIG. 1 in flattened form, a person skilled in the art will appreciate that stent 10 is manufactured for clinical use as an essentially tubular body that may have a variety of shapes, for example, a cylindrical, frustoconical, or hyperboloid shape.

Stent 10 may be produced from a variety of biocompatible materials and may be deployed at a target vessel using techniques also known in the art. For example, stent 10 may be manufactured from a shape memory material such as Nitinol (a nickel-titanium alloy) when stent 10 is structured to self-expand after delivery into the target vessel, or may be manufactured from a plastic or metal material such as stainless steel or cobalt chromium alloys when stent 10 is structured to be expanded by inflating a catheter-mounted balloon on which stent 10 is crimped.

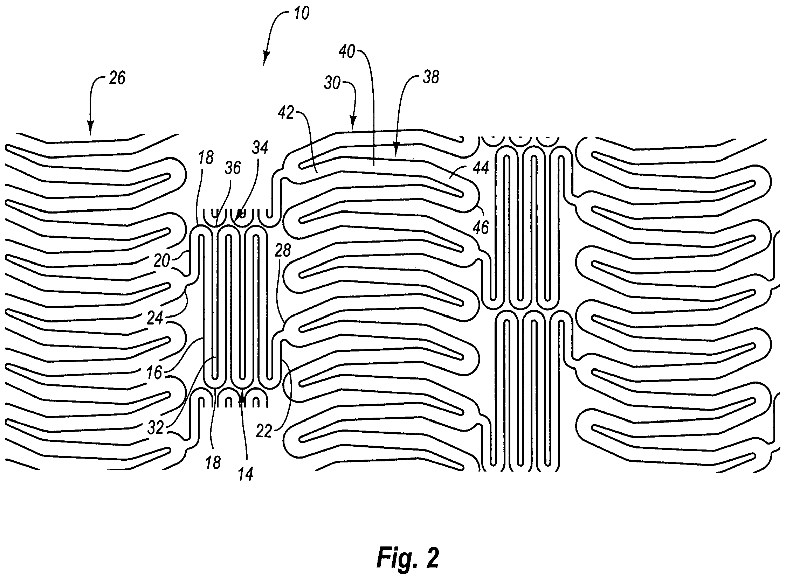

Referring now to FIG. 2, connector 14 includes a plurality of struts 16 that extend circumferentially along the surface of stent 10 in essentially parallel directions. In the illustrated embodiment, five struts 16 of equal length are disposed in circumferential directions and are adjoined sequentially by coupling segments 18, but a higher or lower number of struts 16 (e.g. 2, 3, 4, 6, or 7 and coupling segments 18 (e.g. 3, 4, 5, 7, or 8) may be employed, depending on the distances between the web rings and on the desired density (i.e., on the desired amount of material per surface units) of connector 16.

Coupling segments 18 are shown in FIG. 2 as being arcuate, which is a preferred embodiment of the invention because of the optimal stress distribution of stress in rounded segments in comparison with other segment designs. It should be understood, however, that coupling segments 18 may have a variety of other shapes and that shapes that do not contain points of stress concentration are preferred because the risk of stent rupture is reduced with this type of design.

Connector 14 also includes two struts 20 and 22 of reduced length, which are disposed at opposite ends of connector 14 and which couple connector 14 to neighboring web rings 26 and 30. More particularly, strut 20 joins connector 14 to a first junction bend 24 in first web ring 26 and strut 22 joins connector 14 to a second junction bend 28 in second web ring 30.

First junction bend 24 and second junction bend 28 are shown in FIG. 2 as laterally offset one from the other, that is, not aligned longitudinally. This offset arrangement is preferable over arrangements in which first and second junction bends are longitudinally aligned, because this offset arrangement provides for an improved resistance to foreshortening during stent deployment and as a consequence of bending, compression, elongation and torsion stresses applied to stent 10 after implantation.

While FIG. 2 shows that struts 20 and 22 are coupled to the mid-points of junction bends 24 and 28, in other embodiments of the invention struts 20 and 22 may be coupled to other points of junction bends 24 and 28, for example, struts 20 and 22 may be coupled to (or near) the end points of junction bends 24 and 28, in order to maximize the offset distance between junction bends 24 and 28.

In different embodiments, struts 16 may have different lengths. For example, in the embodiment illustrated in FIG. 2, struts 16 are shown as having the same length as the distance between four junction bends in web ring 26 or 30. In other embodiments of the invention, struts 16 may have a variety of other lengths. Further, FIG. 2 shows struts 16 as essentially rectilinear in shape, but in other embodiments of the invention struts 16 may be each formed by a plurality of segments coupled at different angles (in a fashion similar to the web elements defining web rings 26 and 30) or may even have a curved shape.

In different embodiments, struts 16 may also have a variety of different widths. For example, in the embodiment illustrated in FIG. 2, struts 16 are shown as having widths larger than interstices 32 between struts 16, which provides stent 10 with a high degree of surface density and, accordingly, with elevated scaffolding properties.

A plurality of connectors are aligned circumferentially to couple first web ring 26 to second web ring 30 and the distances between two circumferentially adjacent connectors may vary in different embodiments of the invention. In the embodiment illustrated in FIG. 2, connector 14 is disposed as closely as possible to circumferentially adjacent connector 34, such that coupling segments 36 of connector 34 are nested among coupling segments 18 of connector 14.

The web elements of stent 10 may be shaped as crowns 38 adjoined sequentially by junction bends, as illustrated in FIG. 2. More particularly, each of the web elements or crowns 38 depicted in FIG. 2 is formed by a central member 40, disposed essentially parallel to the longitudinal axis of stent 10 in the contracted configuration of stent 10, and by a first end member 42 and a second end member 44 that extend from opposite ends of central member 40 at two obtuse angles, which may be the same or different.

In the contracted delivery configuration, crowns 38 are nested one into the other. Further, the crowns of neighboring web rings may be disposed in opposite directions, for example, as shown in FIG. 2, the crowns of first web ring 36 may be disposed at 180 degrees in relation to the crowns of second web ring 30. Stents having web elements shaped like the crowns shown in FIG. 2 are described in U.S. Patent Application Publication Nos. US 2004-0193250, 2005-0004651, U.S. Patent Nos. 6,682,554 and 6,602,285, International Publication No. WO 00/13611, and German Patent Publication No. 19840645.

A person skilled in the art will appreciate that web elements of still different shapes may be employed in constructing the web rings and that such alternative designs all fall within the spirit and scope of the present invention. For example, the web elements of web rings 26 and 30 may be shaped like essentially rectilinear struts joined one to the other by arcuate junction bends.

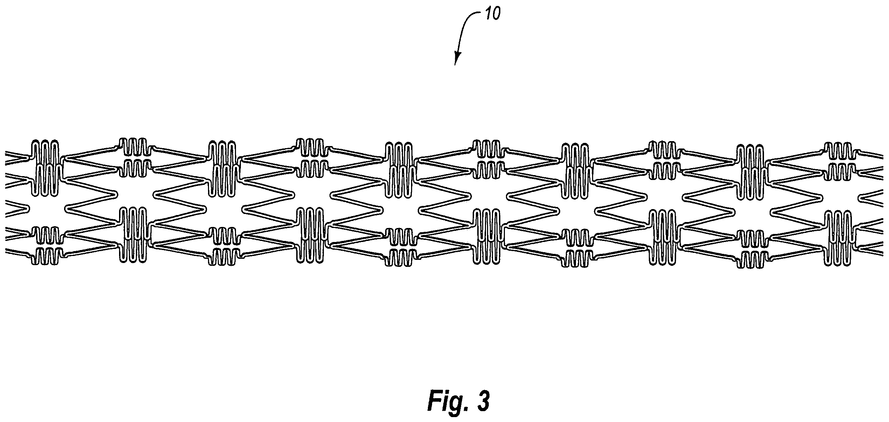

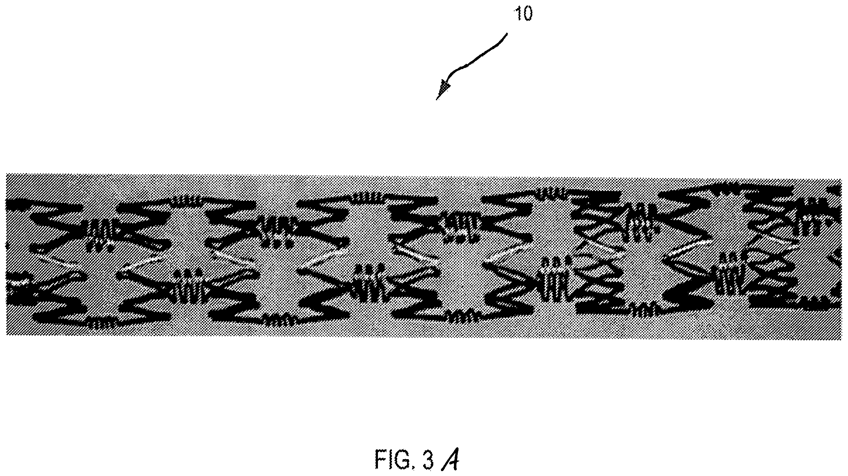

The architecture of stent 10 is particularly stable, as can be seen from the photographs enclosed herein as FIGS. 3-6. Referring first to FIG. 3 and FIG. 3A (the corresponding photo), stent 10 is shown in an at-rest position, without any loads applied thereon. Therefore, FIG. 3 and FIG. 3A depict stent 10 in the configuration described in detail hereinabove, except that FIGS. 1-2 depict stent 10 in a flattened configuration while FIG. 3 and FIG. 3A reproduce stent 10 in a cylindrical, operative configuration.

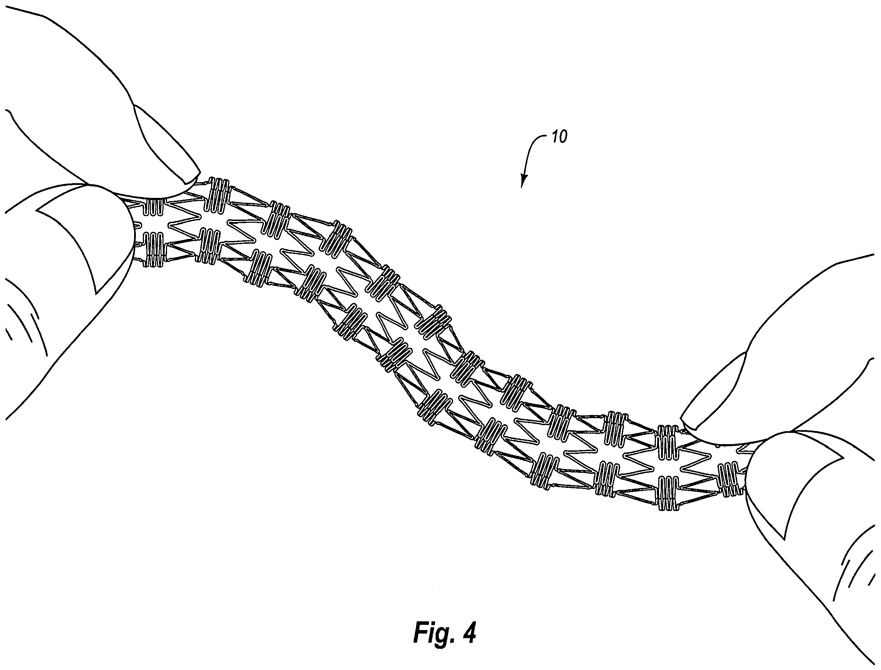

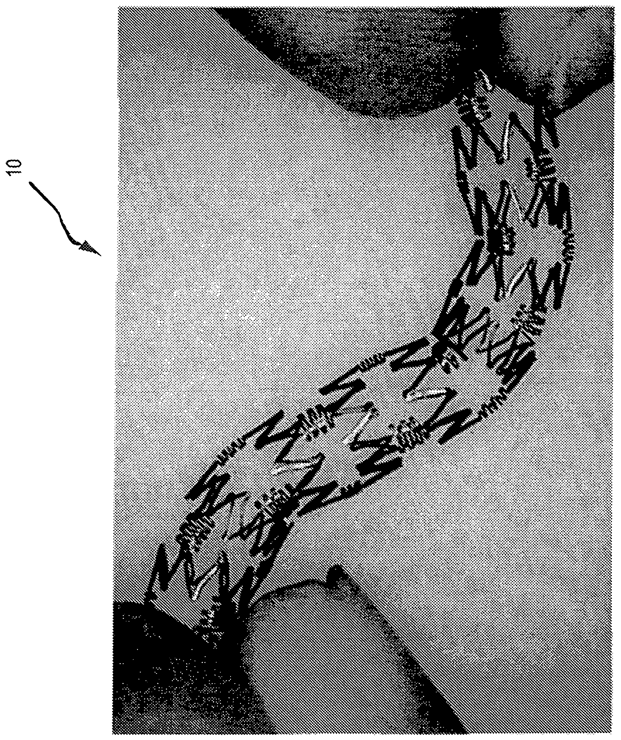

FIG. 4 and FIG. 4A (the corresponding photo) depict stent 10 in a bent configuration. It can be seen that stent 10 still retains its tubular configuration after bending because of the stretching of the connectors (either longitudinally or in fan-like fashion) in the areas of stent 10 under tension, and of the compressing of the connectors (also either longitudinally or in fan-like fashion) in the areas of stent 10 under compression.

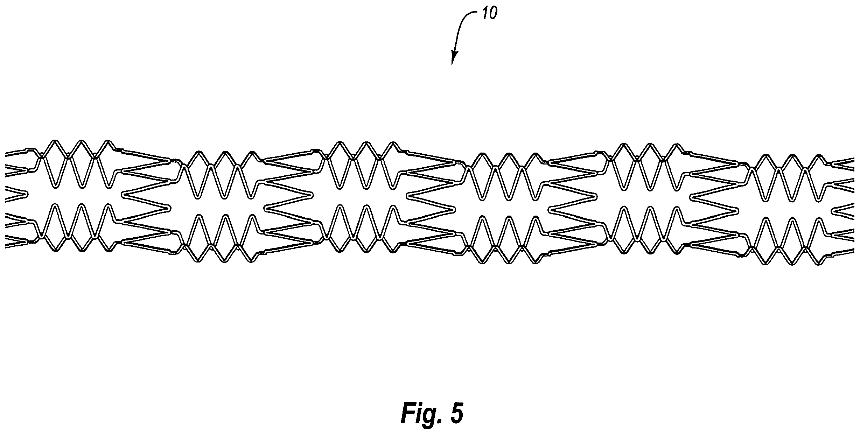

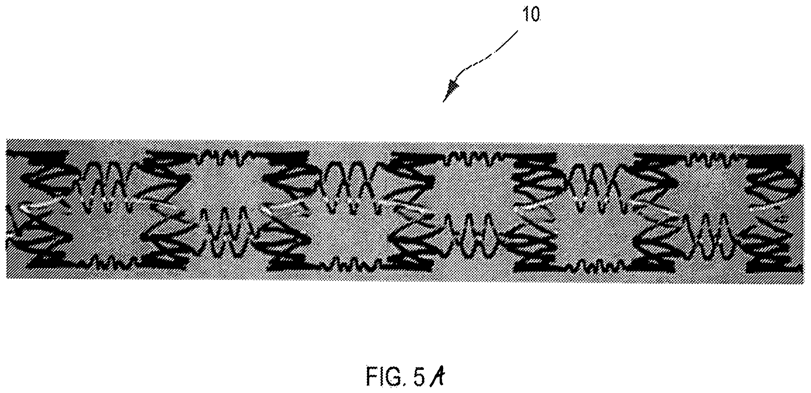

FIG. 5 and FIG. 5A (the corresponding photo) depicts stent 10 in an elongated mode, as a result of tensile forces applied to the longitudinal ends of stent 10. The stable architecture of stent 10 can be seen again, because stent 10 continues to maintain its tubular structure under the applied load.

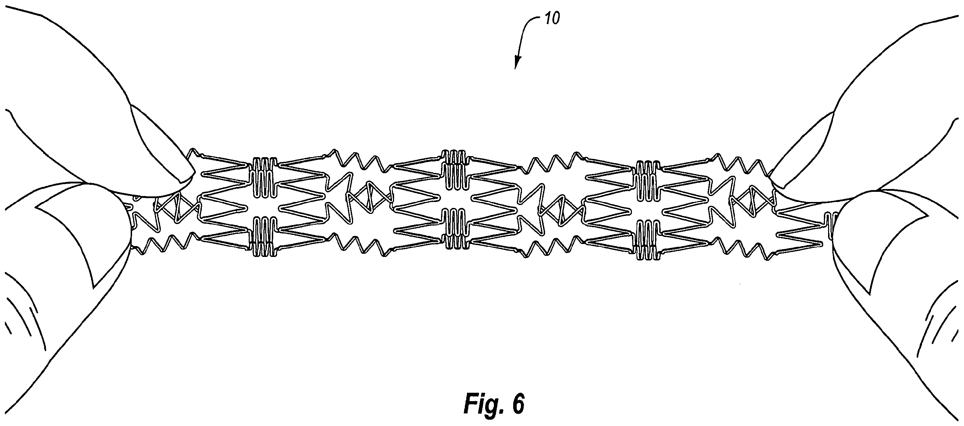

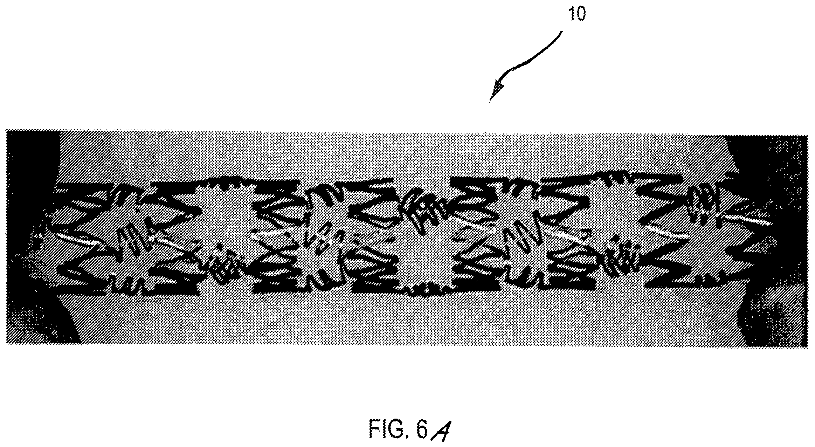

FIG. 6 and FIG. 6A (the corresponding photo) depicts stent 10 when a torsion force is applied to its longitudinal ends. As it can be seen, the construction of stent 10 with connectors configured as described hereinabove and coupled to junction bends that are laterally offset from each other causes the connectors to absorb part or all of the torsional stress transmitted between neighboring web rings, which would otherwise cause the neighboring web rings to rotate one in relation to the other and stent 10 to become deformed, possibly leading to the ultimate fracture of stent 10.

Therefore, an endoprosthesis constructed according to the principles of the present invention will have a stable architecture, preventing traumas to the vessel and to the stent and reducing fractures of the endoprosthesis during service.

The present invention also relates to an endoprosthesis for delivery within a body lumen that includes a plurality of web rings joined one to the other by connectors configured to provide the endoprosthesis with an elevated degree of axial and torsional flexibility and with increased resistance to clinical fatigue. The endoprosthesis of the present invention may be configured as a stent, graft, valve, occlusive device, trocar, or aneurysm treatment device and may be adapted for a variety of intralumenal applications, including vascular, coronary, biliary, esophageal, renal, urological, and gastrointestinal.

In its most basic components, the endoprosthesis of the present invention includes a plurality of web rings coupled one to the other by flexible connectors. Such flexible connectors are formed by struts joined sequentially by foot-shaped extensions, which protrude from the intersections of pairs of the struts and which include an essentially rectilinear segment providing the sole portion of the foot extension and an essentially arcuate segment providing the toe portion of the foot extension.

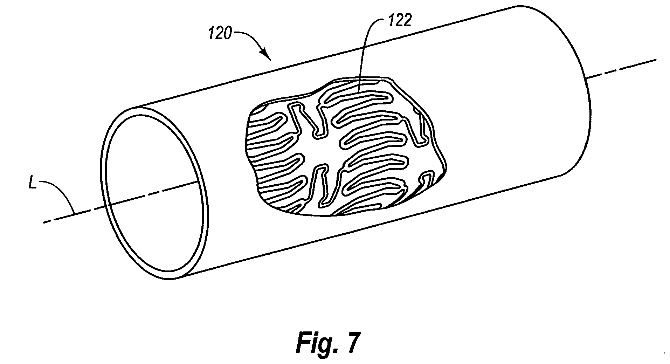

Referring to FIG. 7, an endoprosthesis may be embodied as a stent 120 having a web structure 122 that is expandable from a contracted configuration to an expanded configuration using techniques known in the art. Typically, stent 120 is disposed on a catheter in the contracted configuration and is delivered to a target location where it is expanded. Expansion of stent 120 may be achieved either by inflating a balloon coupled to the catheter or, if stent 120 is manufactured from a shape memory material such as Nitinol (a nickel-titanium alloy), by allowing stent 120 to self-expand until contact with the lumen wall is established. While stent 120 is depicted in FIG. 7 as having an essentially cylindrical shape, stent 120 may be provided with other shapes, for example, with a frustoconical shape or with the shape of a hyperboloid.

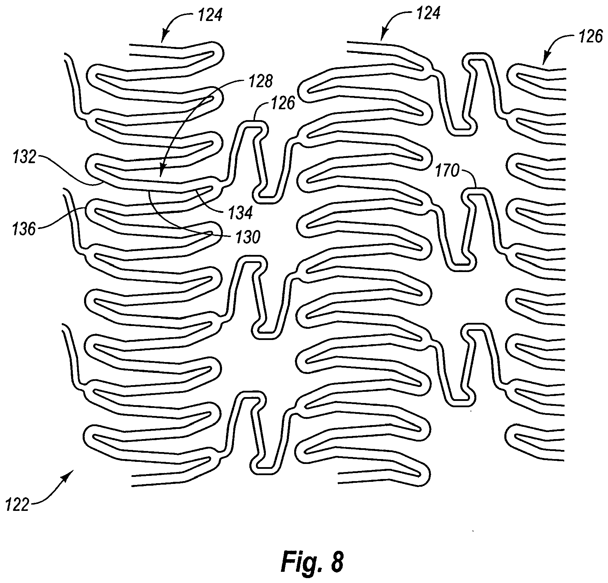

Referring now to FIG. 8, web structure 122 includes a plurality of rings 124 arranged one after the other in the longitudinal direction of stent 120, that is, in a direction parallel to longitudinal axis L of stent 120, and longitudinally coupled one to the other by a plurality of connectors 126.

More particularly, FIG. 8 illustrates web rings 124 in a partially collapsed state. Each of web rings 124 is formed by a plurality of web elements or crowns 128 that are disposed circumferentially around longitudinal axis L and that include a central member 130 and first and second end members 132 and 134 extending respectively from the opposite ends of central member 130. Central member 130 and first and second end members 132 and 134 are each essentially linear in shape, and, when stent 120 is in the contracted delivery configuration, central member 130 is disposed essentially parallel to longitudinal axis L, while first and second members 132 and 134 extend from central member 130 at obtuse angles. Preferably, first and second members 132 and 134 extend from central member 130 at the same angle, but in other embodiments, first and second members 132 and 134 may extend from central member 130 at different angles and may be non-rectilinear in shape.

In the contracted configuration, crowns 128 are nested one into the other and are sequentially adjoined at one end by a junction bend 136 that has an essentially arcuate shape. As shown in FIG. 8, the crowns in one web ring may be disposed with an orientation that is opposite to the orientation of the crowns in a neighboring web ring, in particular, crowns 128 may have an orientation that is 180 degrees different from one web ring 124 to the next. The web rings of the present embodiment are described in U.S. Patent Application Publication Nos. US 2004-0193250, 2005-0004651, U.S. Patent Nos. 6,682,554 and 6,602,285, International Publication Nos. WO 00/13611, and German Patent Publication No. 19840645.

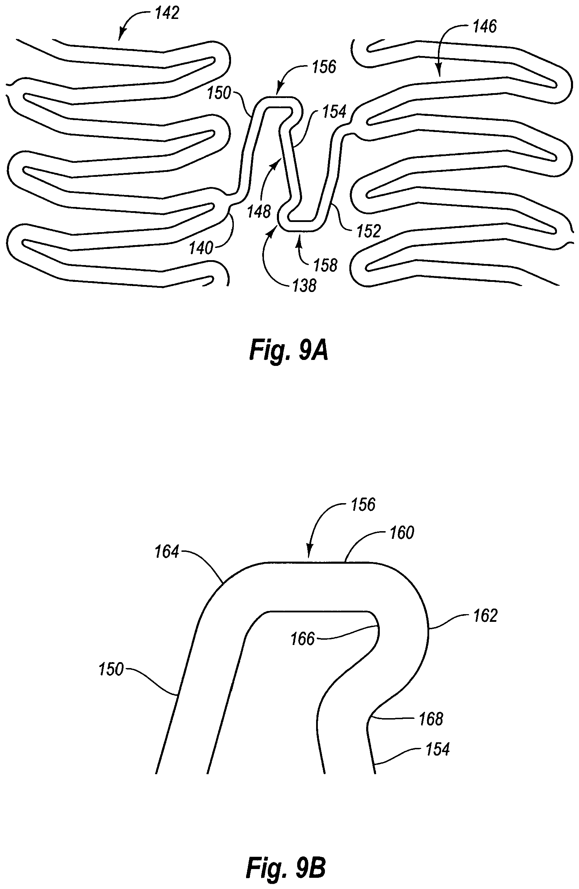

Referring now to FIG. 9A, a connector 138 is shown as coupling a first junction bend 140 in a first web ring 142 to a second junction bend 144 in a second web ring 146. Connector 138 is formed by a plurality of struts 148 adjoined sequentially, more particularly, by three struts 148 that are oriented transversally in relation to longitudinal axis L, for example, by struts 150 and 152 oriented at an angle between about 95 and 105 degrees from longitudinal axis L, and by strut 154 oriented at an angle between about 75 and 85 degrees also from longitudinal axis L. A person skilled in the art will appreciate that connector 138 may include more than three struts 148, and that struts 148 may be disposed at different angles in relation to longitudinal axis L. Moreover, struts 150, 152 and 154 may be disposed at different angles one in relation to the other.

Struts 148 may have a rectilinear profile or have a curved profile, or have a profile defined by a plurality of segments. For example, in the embodiment depicted in FIG. 8, strut 154 has a rectilinear profile and struts 150 and 152 are shown as each having a two-segment profile, with each apex at the intersection of the two segments oriented in the direction of the nearest web ring.

Foot extensions 156 and 158 extend from the intersections between struts 148, and, more particularly, foot extension 156 extends from the intersection between struts 150 and 154, and foot extension 158 extends from the intersection between struts 152 and 154. As shown in the detail view of FIG. 9B related to foot extension 156, but equally applicable to foot extension 158, the foot extension of the present embodiment includes an essentially rectilinear portion extending from strut 150 to provide a sole portion 160, and an arcuate portion connecting sole portion 158 to strut 154 and defining providing a toe portion 162.

Also as shown in FIG. 9B, sole portion 160 is coupled to strut 150 with an area of flexure 164, and toe portion 162 is contoured to provide at least two areas of flexure 166 and 168. Foot extensions 156 and 158 may include straight portions, curved portions or combinations thereof to define an ankle portion, a toe portion, a sole portion and a heel portion. Additionally, sole portion 160 may be a rectilinear segment, as shown in FIG. 9B, or may be contoured as a V-shape, a curved shape, or in a variety of other shapes.

Foot extension 156 may also be provided with a substantially uniform width and thickness throughout. In one variant foot extension 156 has an average width greater than the widths of struts 150 and 154, but if foot extension 156 is provided with an increased width, it may be desirable or necessary to distribute stress or eliminate stress concentrations in the pair of converging struts 150 and 154. For example, at least one or both struts 150 and 154 may be provided with a varied width, or one or both of struts 150 and 154 may be tapered from a width substantially similar to or even greater than that of foot extension 156 to a narrower or larger width. A variety of foot extension designs are disclosed in U.S. Patent No. 7,128,756 to Lowe et al..

Because foot extension 156 generally defines areas of flexure 164, 166 and 168 between the pair of struts 150 and 154, a greater axial and torsional stability and a higher longitudinal flexibility are made possible in comparison with stent designs in which foot extension 156 is absent. Therefore, the structure of connector 138 provides for the absorption of at least some of the axial and torsional deformation of web rings 142 and/or 146, and for the distribution of some of the axial and torsional strain outside of crowns 128. These improved properties over the stents in the prior art provide stent 120 with improved clinical resistance to fatigue.

The multiple areas of flexure of foot extension 156 can also compensate for foreshortening upon deployment of stent 120. As web rings 142 and 146 are expanded, foot extension 156 can adjust or compensate for some or all of the change that occurs in the longitudinal dimensions of those web rings. Similarly, foot extension 156 can be stiffened by increasing the width of one or both of the sole and toe portions 160 and 162, or by otherwise altering the geometry of foot extension 156 in a suitable manner, to reduce the amount in which foot extension 156 opens, and thus reduce the extent of related foreshortening that occurs at the connection location.

In FIG. 9A, foot extensions 156 and 158 have toe portions oriented inwards and facing each other, that is, the toe portion of foot extension 156 is oriented in the direction of web ring 146 and the toe portion of foot extension 158 is oriented in the direction of web ring 142. In other cases, the toe portions of foot extensions 156 and 158 may be both oriented in the same direction, or may still be oriented in opposite directions but outwards, that is, foot extension 156 would face web ring 142 and foot extension 158 would face web ring 146. In addition, foot extensions 156 and 158 may be oriented in directions not parallel to longitudinal axis L and may be disposed in the same direction or in directions diverging one from the other.

Referring again to FIG. 8, not every crown 128 in web ring 124 is coupled to another crown in a neighboring web ring by a connector 126, but only one of every three crowns 128 is so coupled. A person skilled in the art will appreciate that, each crown 128 in one web ring 124 may be coupled to another crown in a neighboring web ring 124, although such design would increase the density of stent 120, correspondingly increasing scaffolding properties in the target location but also decreasing flexibility due to the denser configuration. The above described designs require that the lengths and proportions of struts 148 be dimensioned to achieve the desired nesting among the various connectors 126 when stent 120 is in the contracted delivery configuration.

Connectors 126 coupling a first web ring to a second web ring may not be longitudinally aligned with connectors 170 coupling the second web ring to a third web ring, but instead may be laterally offset one in relation to the other. This arrangement prevents high-density rows of longitudinally aligned connectors from alternating with low-density rows having less material. Instead, the construction of FIG. 8 provides for a more homogenous material distribution along the length of stent 120, which in turn provides for a more homogeneous distribution of stress and strain and reducing the risk of fracture. With this configuration, stent 20 can better retain its tubular shape when bent.

Connectors 170 may be oriented symmetrically with respect to connectors 126, more particularly, connectors 170 may be disposed as mirror images of connectors 126, or alternatively connectors 126 and 170 may be disposed in identical directions, that is with struts and foot extensions oriented in the same directions.

Referring now to FIGS. 8 and 9A, connectors 126 may not couple junction bends that are longitudinally aligned, but may couple junction bends that are laterally offset one from the other. With specific reference to FIG. 9A, connector 156 is shown as coupling not junction bends 140 and 172, which are longitudinally aligned, but instead junction bends 140 and 144 that are laterally offset one from the other. The relative lengths of struts 148 may be adjusted to optimize this offset coupling, for example, central strut 154 may be longer than struts 150 and 152. The radii and curvatures of the toe portions of foot extensions 156 and 158, and the end portions of connector 138 that provide the physical coupling of connector 138 to junction bends 140 and 144, may also be adjusted to optimize such offset coupling and to maintain, for example, a direction of the sole portions of foot extensions 156 and 158 parallel to longitudinal axis L.

Referring now again to FIGS. 7 and 9A, when stent 120 is compressed axially, the offset coupling between junction bends, and, consequently, the offset coupling between the crowns of neighboring web rings 142 and 146 generates a moment that causes a rotational motion of connector 138. Such rotational motion enables connector 138 to engage an adjacent crown when stent 120 is crimped to achieve the contracted delivery configuration, preventing a further compression of the connection segment and improving the deliverability of stent 120.

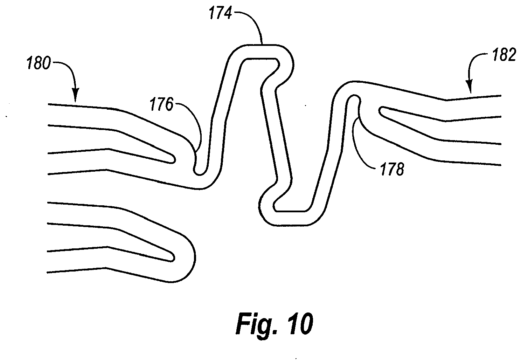

FIG. 9A further shows that connector 138 may be coupled to essentially the midpoint of junction bends 140 and 144. In other cases, connector 138 may be coupled to junction bends 140 and 144 at points other than the respective midpoints. For example, in FIG. 10 a connector 174 is coupled to junction bends 176 and 178 near their respective ends to maximize the distance of the coupling points on junction bends 176 and 178 from a midpoint of connector 174. This arrangement provides for an increase in the moment generated by the offset coupling of web rings 180 and 182 in comparison to the arrangement depicted in FIG. 9A. In other cases, connector 126, as generally shown in FIG. 8, may be coupled to still other points in the designated junction bends, for example, to the points closer to a midpoint of connector 126.

Stent 120 may be manufactured from a variety of biocompatible materials, including metal and plastic materials. For example but not by way of limitation, stent 120 may be manufactured from Nitinol or other shape memory material if a self-expanding configuration of stent 120 is desired, or from stainless steel or a Cobalt Chromium alloy if a balloon expansion is foreseen. Alternatively, stent 120 may be manufactured from a plastic material that enables either a permanent stent placement or a temporary stent placement. For example, stent 120 may be made from a plastic absorbing material.

In some embodiments, crowns 128 and connectors 126 may be manufactured from a biodegradable material when it is expected that only temporary vessel support is required. In another embodiment, only connectors 126 may be manufactured from a biodegradable material, so that the scaffolding provided by stent 120 may change over time by having connectors 126 gradually dissolve in the fluid carried by the vessel (for example, blood), leaving web rings 124 intact so that they may be disposed at specific angles in relation to each other, as required by the patient's anatomy or by the movements of the patient's body.

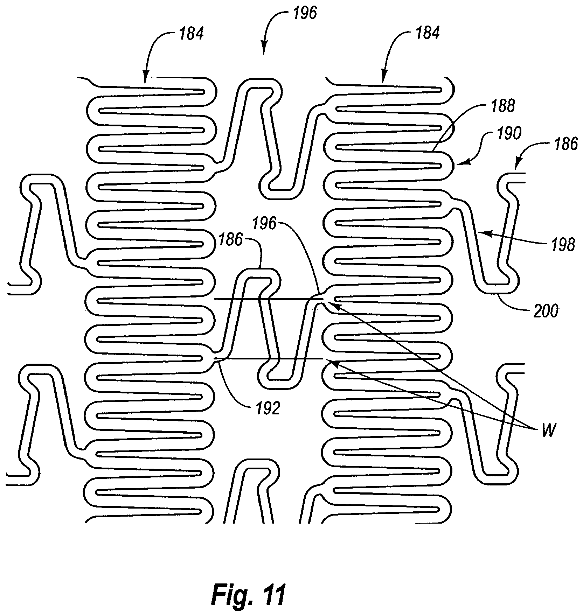

The embodiments described hereinbefore relate to web elements shaped as crowns 128 of FIG. 8. The connector of the present invention is also applicable to stents having web elements with different designs. For example, FIG. 11 illustrates a flat view of a portion of a web structure 196 formed by a plurality of web rings 184 having web elements 188 shaped differently from crowns 128 and coupled by connectors 186.

As shown in FIG. 11, web rings 184 are composed of a plurality of web elements 188 that are each essentially rectilinear and adjoined sequentially by junction bends 190, with junction bends 192 and 194 coupled by connectors 186 being laterally offset by a distance W. The structure and mode of coupling of connectors 186 is the same as previously described with reference to FIGS. 8-10 and will not repeated here for the sake of brevity. Connector 186 is still formed by a plurality of struts 198 connected by foot extensions 200.

A person skilled in the art will appreciate that web elements with different shapes may also be employed in constructing the web rings. A person skilled in the art will also appreciate that the connectors in any of the above described examples may be coupled to junction bends with arcuate segments, for example, segments 192 and 194 in FIG. 11, or with segments of different shapes.

While the invention has been described in connection with the above described embodiments, it is not intended to limit the scope of the invention to the particular forms set forth, but on the contrary, it is intended to cover such alternatives, modifications, and equivalents included within the scope of the invention, which is limited only by the appended claims.

## Claims

1. An endoprosthesis for delivery in a body lumen comprising:
a web structure defining an essentially tubular body expandable from a contracted configuration to an expanded configuration;
a plurality of longitudinally adjacent web rings (12) defining the web structure; and
a plurality of sequentially adjoined web elements (38) defining the web rings (12), pairs of the web elements (38) being sequentially adjoined at junction bends (24, 28),
wherein a first junction bend (24) in a first web ring (12, 26) is coupled to a second junction bend (28) in a second web ring (12, 30) by a connector (14) having a plurality of struts (16) of essentially equal length extending circumferentially,
wherein the struts (16) are essentially parallel and sequentially joined one to the other,
**characterized in that**
the connector (14) consists of five struts (16) of essentially equal length and of two struts (16, 20, 22) of reduced length;
wherein one of the struts (16, 20) of reduced length is coupled to the first junction bend (24) and the other strut (16, 22) of reduced length is coupled to the second junction bend (28); and
wherein the struts (16) are joined one to the other by arcuate coupling segments (18).

2. The endoprosthesis of claim 1, wherein arcuate coupling segments (18) of a first connector (14) are nested between arcuate coupling segments of a second circumferentially adjacent connector (14).

3. The endoprosthesis of claim 1, wherein the first junction bend (24) is laterally offset in relation to the second junction bend (28); or
wherein the interstices between the struts (16) are narrower than the widths of the struts (16).

4. The endoprosthesis of claim 1, wherein the connector (14) spans circumferentially for a distance substantially equal to the spacing between the midpoints of four adjacent junction bends (24, 28) in the second web ring (12, 30).

5. The endoprosthesis of claim 1, wherein the connector (14) couples a midpoint in the first junction bend (24) to a midpoint in the second junction bend (28).

6. The endoprosthesis of claim 1, wherein the connector (14) couples an endpoint in the first junction bend (24) to an endpoint in the second junction bend (28).

7. The endoprosthesis of claim 1, wherein the endoprosthesis is a stent (10).

8. The endoprosthesis of claim 1, wherein the struts (16) are substantially rectilinear in shape.

9. The endoprosthesis of claim 1, wherein the web elements (38) are substantially rectilinear in shape.

10. The endoprosthesis of claim 1,
wherein each of the web elements (38) comprises a central member (40) having a first end and a second end (42, 44),
wherein the central member (40) is disposed essentially parallel to the longitudinal axis in the contracted configuration,
wherein the central member (40) is connected at the first end to a first end member (42) at a first obtuse angle, and
wherein the central member (40) is connected at the second end to a second end member (44) at a second obtuse angle.

11. The endoprosthesis of claim 10, wherein the first and the second obtuse angles are essentially equal.

12. The endoprosthesis of claim 10, wherein the web elements (38) of each web ring (12) are nested one into the other in the contracted delivery configuration, and wherein the junction bends (24, 28) have an arcuate shape.

13. The endoprosthesis of claim 10, wherein the web elements (38) in the first web ring (12, 26) are oriented at approximately 180 degrees in relation to the web elements (38) in the neighboring web ring (12).

14. The endoprosthesis of claim 1, wherein the web structure is configured to self-expand from the contracted configuration to the expanded configuration.

15. The endoprosthesis of claim 1, wherein the web structure is configured to expand from the contracted configuration to the expanded configuration by application of a radial pressure to an interior surface of the essentially tubular body.

## Patentansprüche

1. Endoprothese zur Einbringung in ein Körperlumen umfassend:
eine Stegstruktur, welche einen im Wesentlichen rohrförmigen Körper definiert, welcher von einer zusammengezogenen Gestaltung zu einer expandierten Gestaltung expandierbar ist;
eine Vielzahl von längslaufend benachbarten Stegringen (12), welche die Stegstruktur definieren; und
eine Vielzahl von aufeinanderfolgend verbundenen Stegelementen (38), welche die Stegringe (12) definieren, wobei Paare der Stegelemente (38) an Verbindungsbögen (24, 28) aufeinanderfolgend verbunden sind,
wobei ein erster Verbindungsbogen (24) in einem ersten Stegring (12, 26) mit einem zweiten Verbindungsbogen (28) in einem zweiten Stegring (12, 30) durch einen Verbinder (14) gekuppelt ist, der eine Vielzahl von im Wesentlichen gleiche Länge aufweisenden, sich in Umfangsrichtung erstreckenden Streben (16) besitzt,
wobei die Streben (16) im Wesentlichen parallel verlaufen und aufeinanderfolgend miteinander verbunden sind,
**dadurch gekennzeichnet, dass**
der Verbinder (14) aus fünf Streben (16) von im Wesentlichen gleicher Länge und aus zwei Streben (16, 20, 22) von reduzierter Länge besteht;
wobei eine der Streben (16, 20) von reduzierter Länge mit dem ersten Verbindungsbogen (24) gekuppelt ist, und die andere Strebe (16, 22) von reduzierter Länge mit dem zweiten Verbindungsbogen (28) gekuppelt ist; und
wobei die Streben (16) durch bogenförmige Kupplungssegmente (18) miteinander verbunden sind.

2. Endoprothese nach Anspruch 1, wobei bogenförmige Kupplungssegmente (18) eines ersten Verbinders (14) zwischen bogenförmigen Kupplungssegmenten eines zweiten umfänglich benachbarten Verbinders (14) geschachtelt sind.

3. Endoprothese nach Anspruch 1, wobei der erste Verbindungsbogen (24) in Bezug auf den zweiten Verbindungsbogen (28) seitlich versetzt ist; oder wobei die Spalten zwischen den Streben (16) schmaler sind als die Breiten der Streben (16).

4. Endoprothese nach Anspruch 1, wobei sich der Verbinder (14) umfänglich über einen Abstand erstreckt, der gleich der Distanz zwischen den Mittelpunkten von vier benachbarten Verbindungsbögen (24, 28) in dem zweiten Stegring (12, 30) ist.

5. Endoprothese nach Anspruch 1, wobei der Verbinder (14) einen Mittelpunkt in dem ersten Verbindungsbogen (24) mit einem Mittelpunkt in dem zweiten Verbindungsbogen (28) verkoppelt.

6. Endoprothese nach Anspruch 1, wobei der Verbinder (14) einen Endpunkt in dem ersten Verbindungsbogen (24) mit einem Endpunkt in dem zweiten Verbindungsbogen (28) verkoppelt.

7. Endoprothese nach Anspruch 1, wobei die Endoprothese ein Stent (10) ist.

8. Endoprothese nach Anspruch 1, wobei die Streben (16) eine im Wesentlichen geradlinige Form aufweisen.

9. Endoprothese nach Anspruch 1, wobei die Stegelemente (38) eine im Wesentlichen geradlinige Form aufweisen.

10. Endoprothese nach Anspruch 1,
wobei jedes der Stegelemente (38) ein, ein erstes Ende und ein zweites Ende (42, 44) aufweisendes, zentrales Element (40) umfasst,
wobei das zentrale Element (40) im Wesentlichen parallel zur Längsachse in der zusammengezogenen Gestaltung angeordnet ist,
wobei das zentrale Element (40) am ersten Ende an ein erstes Endelement (42) in einem ersten stumpfen Winkel angeschlossen ist, und
wobei das zentrale Element (40) am zweiten Ende an ein zweites Endelement (44) in einem zweiten stumpfen Winkel angeschlossen ist.

11. Endoprothese nach Anspruch 10, wobei der erste und der zweite stumpfe Winkel im Wesentlichen gleich sind.

12. Endoprothese nach Anspruch 10, wobei die Stegelemente (38) jedes Stegrings (12) in der zusammengezogenen Einbringungs-Konfiguration ineinander geschachtelt sind, und wobei die Verbindungsbögen (24, 28) eine bogenförmige Form aufweisen.

13. Endoprothese nach Anspruch 10, wobei die Stegelemente (38) in dem ersten Stegring (12, 26) in Bezug auf die Stegelemente (38) in dem benachbarten Stegring (12) um ungefähr 180 Grad ausgerichtet sind.

14. Endoprothese nach Anspruch 1, wobei die Stegstruktur so gestaltet ist, dass sie sich von der zusammengezogenen Gestaltung zur expandierten Gestaltung selbst expandiert.

15. Endoprothese nach Anspruch 1, wobei die Stegstruktur so gestaltet ist, dass sie sich von der zusammengezogenen Gestaltung zur expandierten Gestaltung durch Aufbringen eines Radialdrucks auf eine Innenfläche des im Wesentlichen rohrförmigen Körpers expandiert.

## Revendications

1. Endoprothèse pour la délivrance dans une lumière corporelle, comprenant
une structure de bande définissant un corps essentiellement tubulaire, extensible d'une configuration contractée à une configuration dilatée ;
une pluralité d'anneaux de bande longitudinalement adjacents (12) définissant la structure de bande ; et
une pluralité d'éléments de bande séquentiellement adjacents (38) définissant les anneaux de bande (12), des paires d'éléments de bande (38) étant séquentiellement jointes au niveau de courbures de jonction (24, 28),
une première courbure de jonction (24) dans un premier anneau de bande (12, 26) étant couplée à une deuxième courbure de jonction (28) dans un deuxième anneau de bande (12, 30) par un connecteur (14) ayant une pluralité d'entretoises (16) de longueur essentiellement égale s'étendant circonférentiellement,
les entretoises (16) étant essentiellement parallèles et reliées séquentiellement l'une à l'autre,
**caractérisée en ce que**
le connecteur (14) est constitué de cinq entretoises (16) de longueur essentiellement égale et de deux entretoises (16, 20, 22) de longueur réduite ; dans laquelle l'une des entretoises (16, 20) de longueur réduite est couplée à la première courbure de jonction (24) et l'autre entretoise (16, 22) de longueur réduite est couplée à la deuxième courbure de jonction (28) ; et
dans laquelle les entretoises (16) sont jointes l'une à l'autre par des segments de couplage arqués (18).

2. Endoprothèse selon la revendication 1, dans laquelle les segments de couplage arqués (18) d'un premier connecteur (14) sont emboîtés entre des segments de couplage arqués d'un deuxième connecteur circonférentiellement adjacent (14).

3. Endoprothèse selon la revendication 1, dans laquelle la première courbure de jonction (24) est décalée latéralement par rapport à la deuxième courbure de jonction (28) ; ou
dans laquelle les interstices entre les entretoises (16) sont plus étroits que les largeurs des entretoises (16).

4. Endoprothèse selon la revendication 1, dans laquelle le connecteur (14) s'étend circonférentiellement sur une distance essentiellement égale à l'espacement entre les points centraux de quatre courbures de jonction adjacentes (24, 28) dans le deuxième anneau de bande (12, 30).

5. Endoprothèse selon la revendication 1, dans laquelle le connecteur (14) couple un point milieu dans la première courbure de jonction (24) à un point milieu dans la deuxième courbure de jonction (28).

6. Endoprothèse selon la revendication 1, dans laquelle le connecteur (14) couple un point terminal dans la première courbure de jonction (24) à un point terminal dans la deuxième courbure de jonction (28).

7. Endoprothèse selon la revendication 1, dans laquelle l'endoprothèse est un stent (10).

8. Endoprothèse selon la revendication 1, dans laquelle les entretoises (16) ont une forme essentiellement rectiligne.

9. Endoprothèse selon la revendication 1, dans laquelle les éléments de bande (38) ont une forme essentiellement rectiligne.

10. Endoprothèse selon la revendication 1,
dans laquelle chacun des éléments en bande (38) comprend un élément central (40) ayant une première extrémité et une deuxième extrémité (42, 44), dans laquelle l'élément central (40) est disposé essentiellement parallèlement à l'axe longitudinal dans la configuration contractée,
dans laquelle l'élément central (40) est relié au niveau de la première extrémité à un premier élément d'extrémité (42) à un premier angle obtus, et
dans laquelle l'élément central (40) est relié au niveau de la deuxième extrémité à un deuxième élément d'extrémité (44) à un deuxième angle obtus.

11. Endoprothèse selon la revendication 10, dans laquelle les premier et deuxième angles obtus sont essentiellement égaux.

12. Endoprothèse selon la revendication 10, dans laquelle les éléments de bande (38) de chaque anneau de bande (12) sont emboîtés les uns dans les autres dans la configuration de distribution contractée, et dans lequel les courbures de jonction (24, 28) ont une forme arquée.

13. Endoprothèse selon la revendication 10, dans laquelle les éléments de bande (38) du premier anneau de bande (12, 26) sont orientés à environ 180 degrés par rapport aux éléments de bande (38) dans l'anneau de bande voisin (12).

14. Endoprothèse selon la revendication 1, dans laquelle la structure de bande est configurée pour s'étendre automatiquement de la configuration contractée à la configuration dilatée.

15. Endoprothèse selon la revendication 1, dans laquelle la structure de bande est configurée pour s'étendre de la configuration contractée à la configuration dilatée par application d'une pression radiale sur une surface intérieure du corps essentiellement tubulaire.
